(19) Europäisches Patentamt
European Patent Office
Office européen des brevets

(11) EP 0 754 446 A2

(12) **DEMANDE DE BREVET EUROPEEN**

(43) Date de publication:
**22.01.1997 Bulletin 1997/04**

(51) Int Cl.6: **A61K 7/42**, A61K 7/48

(21) Numéro de dépôt: **96401414.6**

(22) Date de dépôt: **26.06.1996**

(84) Etats contractants désignés:
**BE DE ES FR GB IT NL**

(30) Priorité: **20.07.1995 FR 9508818**

(71) Demandeur: **L'OREAL**
**75008 Paris (FR)**

(72) Inventeurs:
- **Simon, Pascal**
**94400 Vitry sur Seine (FR)**
- **Gagnebien, Didier**
**92320 Chatillon (FR)**
- **Leroy, Laurence**
**78120 Rambouillet (FR)**
- **Candau, Didier**
**91570 Bièvres (FR)**

(74) Mandataire: **Lhoste, Catherine**
**L'OREAL,**
**D.P.I.,**
**90 rue du Général Roguet**
**92583 Clichy Cédex (FR)**

(54) **Utilisation d'un agent antioxydant et/ou antiradicalaire dans une composition filtrante à usage cosmétique ou dermatologique**

(57) L'invention se rapporte à l'utilisation d'au moins un agent antiradicalaire et/ou anti-oxydant ne présentant pas de propriété filtrante dans le rayonnement ultraviolet A, dans une composition cosmétique ou dermatologique contenant au moins un filtre ultraviolet A, en vue de protéger contre les rayons ultraviolets A la peau et/ou les cheveux, ce filtre et cet agent agissant en synergie pour conférer à la composition un indice de protection dans ce rayonnement ultraviolet A, supérieur à celui du filtre seul.

EP 0 754 446 A2

## Description

L'invention se rapporte à une composition notamment cosmétique ou dermatologique, ayant des propriétés filtrantes élevées . Elle permet en particulier de prévenir et/ou de lutter contre le vieillissement de la peau notamment dû aux rayonnements ultraviolets A ainsi que de protéger la peau et/ou les cheveux contre ces rayonnements et contre les radicaux libres. Cette composition se présente sous forme d'une crème blanche onctueuse ou d'un gel pouvant être appliqué sur le visage, le corps et/ou les jambes d'êtres humains ainsi que sur les mains et le cuir chevelu.

Au cours du temps, il apparaît différents signes sur la peau, très caractéristiques du vieillissement, se traduisant notamment par une modification de la structure et des fonctions cutanées.

Les principaux signes cliniques du vieillissement cutané sont notamment l'apparition de ridules et de rides profondes, en augmentation avec l'âge. On constate en particulier une désorganisation du "grain" de la peau, c'est-à-dire que le micro-relief est moins régulier et présente un caractère anisotrope.

Par ailleurs, le teint de la peau est généralement modifié ; il apparaît plus pâle et plus jaune ; ceci semble être dû essentiellement à une désorganisation de la microcirculation (moins d'hémoglobine au niveau du derme papillaire). De plus, de nombreuses taches colorées et/ou plus foncées apparaissent à la surface de la peau, et plus spécialement sur les mains, conférant à la peau une hétérogénéité. En général, ces taches sont dues à une production importante de mélanine dans l'épiderme et/ou le derme de la peau. Dans certains cas, ces taches peuvent devenir cancéreuses. Aussi, on cherche de plus en plus à diminuer, voire éliminer, ces taches. Par ailleurs, il peut exister sur certaines zones de la peau des irritations diffuses et parfois des télangiectasies.

Un autre signe clinique du vieillissement est l'aspect sec et rêche de la peau qui est dû essentiellement à une desquamation plus importante ; ces squames, en diffractant les rayons lumineux, participent aussi à l'aspect un peu gris du teint.

On constate enfin une perte de fermeté et de tonicité de la peau qui, comme pour les rides et les ridules, s'explique du moins en partie par une atrophie dermique et épidermique ainsi qu'un applatissement de la formation dermoépidermique ; la peau est moins épaisse et plus flasque, et l'épaisseur de l'épiderme diminue.

On constate donc que les signes cliniques du vieillissement cutané résultent essentiellement d'un dysfonctionnement des principaux mécanismes biologiques intervenant au niveau de la peau.

Ce vieillissement peut être de nature physiologique mais également photoinduit, c'est-à-dire dû à une exposition répétée de la peau à la lumière solaire, et notamment aux rayonnements ultraviolets A. L'action de cette lumière sur les constituants de la peau et sur le sébum sécrété par la peau entraîne en particulier la formation de radicaux libres oxygénés. Or, ces radicaux provoquent des dégats importants, notamment dans les membranes cellulaires (perméabilité des membranes), les noyaux des cellules (mutation par action sur l'ARN ou ADN), et les tissus (nécroses, dégénérescence) ; il est donc nécessaire de protéger la peau contre ces radicaux libres.

Aussi, la composition selon l'invention est une composition apte à prévenir et/ou lutter contre l'apparition du vieillissement et les signes de vieillissement, comme les rides, les ridules, à prévenir et/ou lutter contre les taches de pigmentation de la peau, notamment dues au vieillissement, ainsi qu'à protéger la peau notamment par suppression de la formation de radicaux libres oxygénés.

Pour lutter efficacement contre le vieillissement prématuré de la peau, un des moyens connus est d'utiliser un filtre UVA absorbant entre 320 et 400 nm. Ce dernier permet de diminuer l'excès de radicaux libres photo-induits. Toutefois avec la plupart des compositions contenant des filtres UVA, la protection n'est pas totale. Ainsi, lors d'expositions répétées, la quantité résiduelle de radicaux libres persistant malgré la protection par le filtre UVA, peut provoquer à la longue des phénomènes de vieillissement photo-actinique.

Une solution peut consister à augmenter les quantités de filtres UVA, mais il est peu conseillé d'utiliser des taux trop importants de filtres dans les produits cosmétiques de soin quotidien. En effet, avec la plupart des filtres on atteint souvent un indice de protection maximum qu'il est très difficile d'améliorer en augmentant le taux des filtres. En outre, il faut tenir compte du fait que les produits de soin quotidien sont plus fréquement utilisés que d'autres produits saisonniers contenant des filtres comme les produits solaires par exemple, ce qui peut entraîner des inconforts cosmétiques et des problèmes de toxicité par voie topique.

Pour lutter plus efficacement contre le vieillissement photo-actinique, il est donc intéressant de trouver un autre moyen que celui consistant à utiliser des quantités importantes de filtre UVA. En particulier, il est possible d'associer aux filtres UVA des molécules capables de bloquer les réactions en chaîne des radicaux libres avant les étapes ultimes de dégradation des constituants biologiques de la peau. Ces composés sont des agents anti-oxydants et/ou antiradicaux libres.

On sait que les radicaux libres photo-induits proviennent en grande partie de l'oxygène moléculaire. Etant donné qu'il est abondant dans l'organisme et qu'il a une forte capacité à accepter les électrons, les radicaux libres et les espèces activées de l'oxygène qui en dérivent sont les plus nombreux à participer à des réactions radicalaires. On peut dénombrer :

L'oxygène singulet : non radicalaire, très oxydant, très toxique et très rare car d'une durée de vie très courte. Il est

le produit de l'excitation de l'oxygène moléculaire par les photons de la lumière.

Le radical anion superoxyde : il est le produit de l'addition d'un électron sur l'oxygène moléculaire. Il peut donner lieu à la production de radicaux libres très réactifs : les radicaux hydroxyles.

Le peroxyde d'hydrogène : non radicalaire mais qui peut donner lieu à la production de radicaux hydroxyles.

Le radical hydroxyle : il est très oxydant donc très réactif et le plus toxique pour les cellules.

On citera en outre les radicaux lipo-peroxydes qui sont les produits d'oxydation des lipides membranaires.

On citera également le fer extracellulaire qui, en réagissant avec le peroxyde d'hydrogène et le radical anion superoxyde accumulés en dehors de la cellule, va favoriser la production du radical hydroxyle.

Pour lutter efficacement contre les radicaux libres oxygénés et donc contre le photovieillissement, la demanderesse a recherché une association d'un filtre UVA avec une ou plusieurs molécules anti-oxydantes et/ou antiradicalaires, présentant un effet de synergie et notamment un indice de protection supérieur à celui du filtre seul alors que ces molécules anti-oxydantes et/ou antiradicalaires ne présentent aucune activité de filtration du rayonnement UVA.

De façon surprenante, la demanderesse a trouvé qu'en associant une ou plusieurs des molécules ci-dessus avec un filtre UVA, on augmentait le pouvoir filtrant de ce dernier et en particulier son indice de protection (IP).

Le pouvoir filtrant dans le domaine des UVA est représenté par l'indice de protection, noté IP. La détermination de cet indice de protection UVA est basée sur la méthode d'évaluation de la pigmentation immédiate et persistante induite par les UVA (Persistant Pigment Darkening : PPD), décrite par Chardon et col. (Method for the UVA protection assessment of sunscreens based on residual immediate pigment darkening. 20th Annual Meeting of the American Society for Photobiology, Marco island, Florida (USA), June 20-24, 1992).

Certes, divers documents décrivent l'association de filtre et de molécules anti-oxydantes et/ou antiradicalaires. Ainsi, il est connu par le document US-A-4603046 d'utiliser la troxerutine pour améliorer le pouvoir de protection et d'hydratation d'une composition solaire contenant un filtre UV. Toutefois, ce document décrit que la troxerutine a des propriétés filtrantes ; elle ne fait donc pas partie des molécules indiquées ci-dessus.

Par ailleurs, il est connu par les documents suivants, des compositions solaires contenant en association un filtre UV et un agent anti-oxydant et/ou antiradicalaire n'ayant pas de propriété filtrante :

Le document FR-A-2687572 se rapporte à l'utilisation de flavonoïdes pour protéger la peau contre l'oxygène singulet, éventuellement dans des compositions solaires.

Le document EP-A-579078 décrit une composition solaire contenant un système antiradicaux libres à base de vitamine E et de 2-(dihydroéthyl)-2-hydroxy-6,10,14-triméthyl-pentadecane dont la présence améliore la pénétration ou la diffusion de la vitamine E.

Le document GB-A-2195244 décrit une composition solaire à base d'un dérivé de la rutine. Grâce à ce dérivé, la composition est photostable.

Le document EP-A-601698 se rapporte à une composition topique à base de tocophérol-ascorbate-phosphate et de filtres pour empêcher le vieillissement de la peau en inhibant la réticulation du collagène cutané.

Le document EP-A-280606 se rapporte à un système anti-oxydant à base d'ester d'ascorbyle, d'un complexant et d'un thiol, éventuellement associés à du tocophérol ou de l'acide caféique.

Le document FR-A-2666226 décrit une composition améliorée pour protéger la peau des radiations, comprenant l'association d'un filtre, d'un composant antiradicalaire et d'un composant anti-élastasique.

Le document FR-A-2708851 se rapporte à une composition cosmétique contenant des nanopigments et un agent anti-oxydant dont la présence empêche la peroxydation des lipides.

Le document JP-A-01-135887 décrit une composition solaire comprenant un dérivé de porphyrine et un chélateur, dont l'association permet d'éviter la dégradation photochimique du squalène de la peau et donc le vieillissement cutané.

Le document JP-A-61-215311 décrit une composition topique comprenant un filtre et un chélateur, ce dernier évitant la dégradation du produit.

Le document WO-A-94/04128 enseigne d'utiliser un chélateur pour traiter l'immunosuppression induite par radiation UVB.

Le document WO-A-94/04131 décrit une composition solaire photostable comprenant un filtre UVB et un filtre UVA, et pouvant contenir les adjuvants habituels tels que des anti-oxydants.

Toutefois, ces divers documents n'enseignent pas que les agents anti-oxydants et/ou antiradicalaires peuvent améliorer l'indice de protection d'un filtre UVA.

Par ailleurs, le document EP-A-671160 se rapporte à l'utilisation d'acides sulfoniques comme agents pour lutter contre le vieillissement intrinsèque de la peau. Ce document ne concerne pas la protection solaire.

Le document EP-A-313305 décrit une composition photoprotectrice contenant des chélateurs ayant des propriétés filtrantes. Ce document ne suggère pas que des chélateurs sans propriétés filtrantes puissent élever l'indice de protection solaire des filtres UVA.

Le document DE-U-9104777 décrit une association de polyol, d'aminoacide et de chélateur, qui améliore la stabilité des compositions topiques, dont les compositions solaires. Toutefois, il n'enseigne pas que cette association puisse avoir une influence sur l'indice de protection solaire.

La présente invention a pour objet une composition à application topique, destinée notamment à prévenir et/ou lutter contre le vieillissement de la peau et/ou les taches cutanées et/ou protéger la peau et/ou les cheveux contre les rayons ultraviolets et/ou contre les radicaux libres, présentant une efficacité supérieure à celle de l'art antérieur.

Ainsi, l'invention a pour objet l'utilisation d'au moins un agent anti-oxydant et/ou antiradicalaire ne présentant pas de propriété filtrante dans le rayonnement ultraviolet A, pour conférer à une composition contenant, dans un milieu acceptable pour une application topique, au moins un filtre absorbant au moins le rayonnement ultraviolet A, un indice de protection (IP) dans ce rayonnement ultraviolet A, supérieur à celui du filtre seul.

Avantageusement, l'agent anti-oxydant et/ou antiradicalaire est un chélateur de fer.

De préférence, la composition de l'invention contient un ou plusieurs autres agents anti-oxydants et/ou antiradicalaires, choisis parmi les agents antilipo-peroxyde, les composés régénérateurs de vitamine E oxydé, les agents antiradical hydroxyle, les agents anti-oxygène singulet et les agents antiradical anion superoxyde et leurs associations.

La composition contient un ou plusieurs filtres UVA en une quantité efficace pour filtrer ce rayonnement UVA. En pratique, ces filtres représentent de 0,01% à 10% de préférence de 0,1% à 5% du poids total de la composition. Ces filtres sont en particulier choisis parmi les filtres organiques ou parmi les pigments qui peuvent être sous forme de nanoparticules (nanopigments) ou non.

Comme pigments, on peut citer notamment le dioxyde de titane ou l'oxyde de zinc, de préférence sous forme de nanopigments. On appelle « nanopigments » les pigments de diamètre moyen inférieur à 100 nm, et de préférence allant de 5 à 50 nm. Ces pigments et nanopigments peuvent être enrobés ou non enrobés.

Comme filtres organiques, on peut utiliser notamment des molécules à une ou plusieurs fonctions sulfoniques éventuellement neutralisées ou des molécules à une ou plusieurs fonctions sulfonates.

Les bases utilisées pour neutraliser les fonctions sulfoniques des filtres UVA sont de préférence des bases organiques comme la triéthanolamine et l'éthylène diamine.

Les filtres UVA utilisables dans l'invention peuvent être lipophiles ou mieux hydrophiles. Comme filtres utilisables dans l'invention, on peut citer les dérivés sulfonés ou sulfonatés de la benzophénone comme l'acide hydroxy-2-méthoxy-4-benzophénone-5-sulfonique, commercialisé notamment par BASF sous le nom d'Uvinul MS-40, ainsi que les dérivés sulfoniques ou sulfonatés du benzylidène camphre.

En particulier, les dérivés de benzylidène camphre utilisables dans l'invention présente la formule générale (a) suivante :

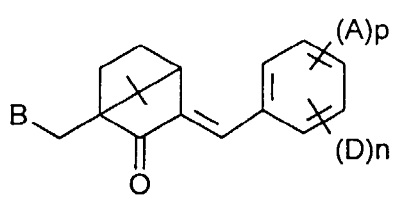

(a)

dans laquelle :

B représente -H ou $-SO_3H$,

$0 \leq p \leq 1$ avec B = $-SO_3H$ quand p = 0,

$0 \leq n \leq 4$,

D représente un ou plusieurs radicaux alkyle ou alcoxy, identiques ou différents quand $n \geq 2$, linéaires ou ramifiés contenant de 1 à 18 atomes de carbone environ, un radical halogéno, un radical hydroxyle.

A, de préférence en méta ou en para, représente

soit un radical $-SO_3H$ ;

soit un groupement :

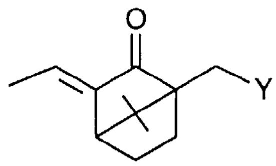

dans lequel Y représente -H ou $-SO_3H$ ;

soit un groupement :

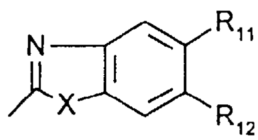

dans lequel:

$R_{11}$ désigne un atome d'hydrogène, un radical alkyle ou alcoxy, linéaire ou ramifié contenant de 1 à 6 atomes de carbone environ ou le radical $-SO_3H$, $R_{11}$ étant $-SO_3H$ lorsque B = -H,

$R_{12}$ désigne un atome d'hydrogène, un radical alkyle ou alcoxy, linéaire ou ramifié, contenant de 1 à 6 atomes de carbone environ,

X est un atome d'oxygène, de soufre ou un groupement -NR-, R étant un atome d'hydrogène ou un radical alkyle, linéaire ou ramifié, contenant de 1 à 6 atomes de carbone environ,

et dans laquelle au moins une fonction $-SO_3H$ est éventuellement neutralisée.

La neutralisation d'une ou plusieurs fonctions peut être obtenue à l'aide d'une base généralement utilisée dans le domaine cosmétique comme la soude, la triéthanolamine, la potasse.

On peut citer comme exemples particuliers de composés de formule (a) les dérivés de formules (I), (II), (III) suivantes :

*Formule (I) :*

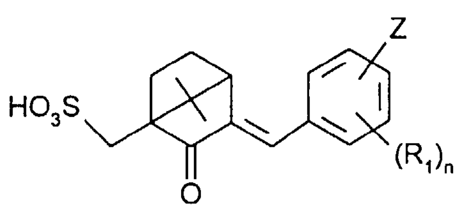

(I)

dans laquelle :

- Z, de préférence en position para ou méta, désigne un groupement

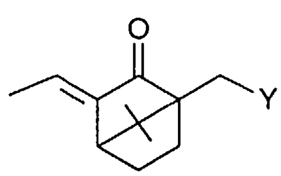

dans lequel Y représente -H ou -$SO_3H$, éventuellement neutralisé,

- n est égal à 0 ou est un nombre allant de 1 à 4 ($0 \leq n \leq 4$),
- $R_1$, représente un ou plusieurs radicaux alkyle ou alkoxy, identiques ou différents, linéaires ou ramifiés, contenant de 1 à 4 atomes de carbone environ.

Un composé de formule (I) particulièrement préféré est celui correspondant à n = 0 , Z en position para et Y = -$SO_3H$ : l'acide benzène 1,4 [di(3-méthylidènecampho 10-sulfonique)], appelé aussi (selon la nomenclature CTFA - 5ème édition) l'acide téréphtalylidène - di-camphosulfonique.

*Formule (II) :*

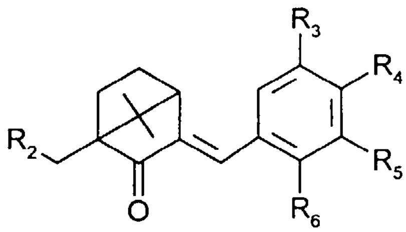

(II)

dans laquelle :

- $R_2$ désigne un atome d'hydrogène ou un radical -$SO_3H$,

- $R_3$, $R_4$ $R_5$ et $R_6$, identiques ou différents, représentent un groupement hydroxyle, un radical alkyle ayant de 1 à 4 atomes de carbone environ, linéaire ou ramifié, un radical alcényle ayant de 2 à 4 atomes de carbone environ, linéaire ou ramifié, un radical alcoxy ayant de 1 à 4 atomes de carbone, linéaire ou ramifié, un radical alcényloxy ayant de 2 à 4 atomes de carbone, linéaire ou ramifié, un radical halogéno ; de plus un radical $R_3$ à $R_6$ seulement peut être un radical -$SO_3H$, au moins un des radicaux $R_3$ à $R_6$ désignant le radical -$SO_3H$ quand $R_2$ est un atome d'hydrogène. Une ou plusieurs fonctions -$SO_3H$ peuvent aussi être neutralisées.

On peut citer comme exemples particuliers les composés suivants de formule (II) dans laquelle :

- $R_4$ désigne le radical -$SO_3H$ en position para du benzylidènecamphre et $R_2$, $R_3$, $R_5$ et $R_6$ désignent chacun un atome d'hydrogène, c'est-à-dire le 4'-sulfo 3-benzylidènecamphre.

- $R_3$, $R_4$, $R_5$ et $R_6$ désignent chacun un atome d'hydrogène et $R_2$ désigne un radical -$SO_3H$, c'est-à-dire l'acide 3-benzylidène campho-10 sulfonique.

- $R_4$ désigne un radical méthyle en position para du benzylidènecamphre, $R_5$ un radical -$SO_3H$ et $R_2$, $R_3$ et $R_6$ représentent un atome d'hydrogène, c'est-à-dire le 4'-méthyl 3'-sulfo 3-benzylidènecamphre.

- $R_4$ désigne un atome de chlore en position para du benzylidènecamphre, $R_5$ un radical -$SO_3H$ et $R_2$, $R_3$ et $R_6$ représentent un atome d'hydrogène, c'est-à-dire le 4'-chloro 3'-sulfo 3-benzylidènecamphre.

- $R_4$ désigne un radical méthyle en position para du benzylidènecamphre, $R_3$, $R_5$ et $R_6$ désignent un atome d'hydrogène et $R_2$ désigne un radical -$SO_3H$, c'est-à-dire l'acide 4'-méthyl 3-benzylidène campho 10-sulfonique.

- $R_2$ représente un radical -$SO_3H$, $R_3$ est un radical méthyle, $R_4$ un atome d'hydrogène, $R_5$ un radical tertiobutyle, $R_6$ un radical hydroxyle, c'est-à-dire l'acide 3'-t-butyl 2'-hydroxy 5'-méthyl-3-benzylidène campho-10-sulfonique.

- $R_2$ représente un radical -$SO_3H$, $R_3$ est un radical méthoxy, $R_4$ un atome d'hydrogène, $R_5$ un radical tertiobutyle, $R_6$ un radical hydroxyle, c'est-à-dire l'acide 3'-t-butyl 2'-hydroxy 5'-méthoxy-3-benzylidène campho-10-sulfonique.

- $R_2$ représente un radical -$SO_3H$, $R_3$ et $R_5$ désignent chacun un radical tertiobutyle, $R_4$ un radical hydroxyle, $R_6$ un atome d'hydrogène, c'est-à-dire l'acide 3',5'-diterbutyl 4'-hydroxy-3-benzylidéne campho-10-sulfonique.

- $R_4$ représente un radical méthoxy en para, $R_5$ représente -$SO_3H$, les radicaux $R_2$, $R_3$ et $R_6$ représentent H, c'est-à-dire le 4'-méthoxy 3'-sulfo-3-benzylidène camphre.

- $R_2$ désigne un radical $-SO_3H$, $R_3$ et $R_6$ représentent H, $R_4$ et $R_5$ formant un radical méthylènedioxy, c'est-à-dire l'acide 3',4'-méthylènedioxy-3-benzylidène campho-10-sulfonique.
- $R_2$ représente un radical $-SO_3H$, $R_4$ un radical méthoxy et les radicaux $R_3$, $R_5$ et $R_6$ représentent H, c'est-à-dire l'acide 4'-méthoxy-3-benzylidène campho-10-sulfonique.
- $R_2$ représente un radical $-SO_3H$, $R_4$ et $R_5$ sont tous deux un radical méthoxy et les radicaux $R_3$ et $R_6$ représentent H, c'est-à-dire l'acide 3',4'-diméthoxy-3-benzylidène campho-10-sulfonique.
- $R_2$ représente un radical $-SO_3H$, $R_4$ est un radical n-butoxy et les radicaux $R_3$, $R_5$ et $R_6$ représentent un atome d'hydrogène, c'est-à-dire l'acide 4'-n.butoxy-3-benzylidène campho-10-sulfonique.
- $R_2$ représente un radical $-SO_3H$, $R_4$ est un radical n-butoxy, $R_5$ est un radical méthoxy et $R_3$ et $R_6$ désignent tous deux un atome d'hydrogène, c'est-à-dire l'acide 3'- méthoxy 4'-n.butoxy-3- benzylidène campho-10-sulfonique.

*Formule (III) :*

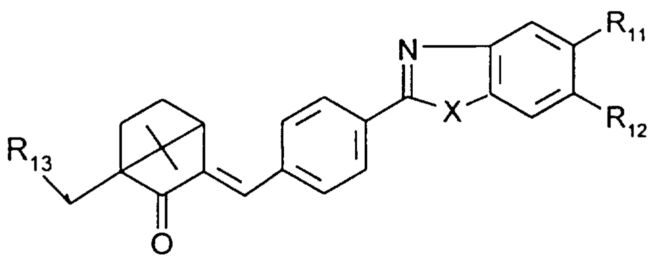

(III)

dans laquelle :

- $R_{11}$ désigne un atome d'hydrogène, un radical alkyle ou alcoxy, linéaire ou ramifié, contenant de 1 à 6 atomes de carbone environ ou un radical $-SO_3H$,
- $R_{12}$ désigne un atome d'hydrogène, un radical alkyle ou alcoxy, linéaire ou ramifié, contenant de 1 à 6 atomes de carbone environ,
- $R_{13}$ désigne un atome d'hydrogène ou un radical $-SO_3H$,
- l'un au moins des radicaux $R_{11}$ et $R_{13}$ désignant un radical $-SO_3H$,
- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer comme exemple particulier de composé de formule (III) : le composé dans lequel X désigne un radical -NH-, $R_{11}$ désigne un radical $-SO_3H$, $R_{12}$ et $R_{13}$ désignent tous deux un atome d'hydrogène, c'est-à-dire l'acide 2-[4-(camphométhylidène) phényl] benzimidazole-5-sulfonique.

Les composés de structures (I), (II), (III) sont décrits dans le brevet US 4.585.597 et les brevets FR 2.236.515, 2.282.426, 2.645.148, 2.430.938, 2.592.380.

On peut citer comme autres exemples de dérivés du benzylidène camphre utilisables dans l'invention les composés de formule générale (b) suivante :

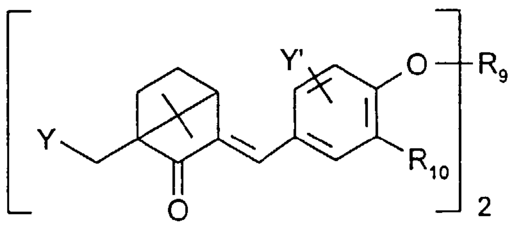 (b)

dans laquelle :

- $R_9$ désigne un radical divalent : $-(CH_2)_m-$ ou $-CH_2-CHOH-CH_2-$ , m étant un nombre entier allant de 1 à 10 ($1 \leq m \leq 10$),
- $R_{10}$ désigne un atome d'hydrogène, un radical alcoxy contenant de 1 à 4 atomes de carbone environ ou un radical divalent - O - relié au radical $R_9$ lorsque celui-ci est divalent lui aussi,
- Y et Y' désignent un atome d'hydrogène ou un radical $-SO_3H$, au moins un de ces radicaux Y ou Y' étant différent de l'hydrogène. Là encore la fonction $-SO_3H$ peut être neutralisée.

On peut citer comme exemples particuliers les composés suivants de formule (b) dans laquelle Y représente $-SO_3H$, Y' est -H, $R_{10}$ est H et $R_9$ est $-CH_2-CH_2-$, c'est à dire l'acide éthylène bis [(4'-oxy benzylidène) 3-campho-10 sulfonique].

Le ou les chélateurs de fer ont de façon avantageuse une constante d'association avec les ions ferreux ou ferriques supérieure à $10^2$, de préférence supérieure à $10^6$. Comme chélateur de fer, on peut citer les sels de l'acide éthylène di-amine tétraméthylène phosphonique et notamment les sels pentasodique, l'éthylène di-amine tétra-acétique et ses sels, l'acide diéthylènediamine pentacétique et son sel pentasodique. On peut citer aussi l'acide citrique, l'acide tartrique et leurs sels de sodium, l'acide phytique, le dibenzyldithiocarbamate. Ces chélateurs sont utilisés à raison de 0,001 % à 5 % et de préférence de 0,1 % à 2 % du poids total de la composition.

On peut aussi utiliser un ou plusieurs agents anti-lipoperoxyde en une quantité allant de préférence de 0,05% à 5% et mieux de 0,2% à 3% du poids total de la coomposition. Ces agents sont par exemple la Vitamine E (ou alpha D,L tocophérol) et ses dérivés, ou tout autre composé efficace in vitro au test dit de Bêta-carotène ou au test du rancimat, comme des extraits naturels tels que les oligomères procyannidoliques (OPC) d'aubépine, de pin ou de raisin, les extraits de noix d'alep, de romarin, le pidolate de lysine, l'éthoxyquine.

Selon l'invention, on peut, en outre, utiliser un ou plusieurs composés régénérateurs de la vitamine E oxydée (produit de la réaction de la Vitamine E avec un radical lipo-peroxyde) tels que la vitamine C ou ses dérivés stabilisés comme l'ascorbyl glucoside, le pidolate de lysine ou d'arginine ou le lauroyl méthionate de lysine. Ces composés peuvent être utilisés par exemple à une concentration allant de 0,01% à 5% de préférence de 0,1% à 1% du poids total de la composition.

On peut utiliser, en outre, un ou plusieurs agents anti-radical hydroxyle tels que la caféine et ses dérivés, ou tout autre composé efficace dans le test dit de l'éthylène, comme le mannitol, le gingko, la carnosine, la taurine, la L-histidine-béta alanine (dipeptide), les extraits de thym. Ces agents peuvent être présents à raison de 0,001% à 5% de préférence de 0,005% à 2% du poids total de la composition.

Un ou plusieurs agent anti-oxygène singulet peuvent, aussi être ajoutés au agents ci-dessus à raison par exemple de 0,0001% à 1% de préférence de 0,0005% à 1% du poids total de la composition. Comme agent anti-oxygène singulet, on peut citer l'éthoxyquine par exemple ou tout autre composé efficace dans le test dit de squalène en présence d'un photosensibilisant comme l'hématoporphyrine. On citera l'algaxan red, la santoquin, les extraits de romarin, et les flavonoïdes comme l'alpha glycosyl rutine.

Un ou plusieurs agents antiradical anion superoxyde peuvent, enfin, être présents, tels que les superoxyde dismutases (SOD[Cu-Zn], SOD[Mn]) ou tout autre composé équivalent ayant une activité in vitro dans le test dit de xanthine/xanthine oxydase, comme les extraits de thé vert et l'héliogénol. Ces agents peuvent représenter de de 0,1 % à 5 % et mieux de 0,5 % à 3 % du poids total de la composition.

Comme SOD, on peut notamment citer les SOD d'origine animale, humaine (par exemple placentaire ou sanguine), végétale (par exemple extraites de champignons, algues, épinards, etc ), ou les SOD extraites de microorganismes (bactéries ou levures), ou encore les SOD recombinantes obtenues par génie génétique. On utilise de préférence les

SOD d'origine végétale ou recombinantes.

De façon avantageuse, la composition de l'invention contient au moins un filtre UVA tel que l'acide benzène 1,4 [di(3-méthylidène-campho 10-sulfonique)], un chélateur de fer tel que le Dequest 2046 et un agent antilipo-peroxyde comme la vitamine E.

La composition de l'invention peut se présenter sous toutes les formes galéniques normalement utilisées pour une application topique telles que les solutions, les gels aqueux ou hydroalcooliques, les émulsions huile-dans-eau ou eau-dans-huile, et plus particulièrement les gouttelettes d'huile dispersées par des sphérules dans une phase aqueuse. Ces sphérules peuvent être des nanoparticules polymériques telles que les nanosphères et les nanocapsules ou mieux des vésicules lipidiques. La composition de l'invention peut se présenter sous forme d'une crème, d'une pommade, d'une lotion ou d'un sérum.

Les huiles utilisables dans l'invention sont celles généralement utilisées dans les domaines concernés. Elles peuvent être végétales, minérales ou synthétiques, éventuellement siliconées et/ou fluorées.

L'invention peut également contenir des adjuvants hydrophiles ou lipophiles comme des gélifiants, des conservateurs, des opacifiants, des émulsionnants, des coémulsionnants, des neutralisants, des parfums et leurs solubilisants ou peptisants, des matières colorantes comme les colorants et les pigments, des charges, ainsi que des actifs lipophiles ou hydrophiles autres que les agents anti-oxydants et/ou antiradicaux libres et que les filtres chimiques absorbant dans l'ultraviolet A. En particulier, il est possible d'adjoindre à la composition des filtres UVB en vue d'éviter les coups de soleil lors de l'exposition des utilisateurs de cette composition aux rayons solaires.

Les quantités d'huile et d'eau sont généralement celles utilisées dans les domaines considérés et sont fonction de la forme galénique de la composition. Pour une émulsion huile-dans-eau ou une dispersion d'huile dans de l'eau par des sphérules lipidiques, l'huile peut représenter de 0,5% à 60% de préférence de 2 % à 40 % du poids total de la composition.

De même les adjuvants sont utilisés en quantité habituelle et peuvent représenter, au total, de 0,1 % à 20 % en poids. Leur quantité est fonction de leur nature.

La composition de l'invention peut être appliquée par voie topique sur toutes les parties du corps et du visage, y compris sur le cuir chevelu, les jambes et les mains.

L'invention a aussi pour objet l'utilisation d'une composition contenant, dans un milieu acceptable pour une application topique, au moins un filtre absorbant au moins le rayonnement ultraviolet A et au moins un agent anti-oxydant et/ou antiradicalaire ne présentant pas de propriété filtrante dans le rayonnement ultraviolet A et conférant à cette composition un indice de protection (IP) dans ce rayonnement ultraviolet A, supérieur à celui du filtre seul, pour le traitement cosmétique des rides et/ou les ridules de la peau induites par un rayonnement ultraviolet A ainsi que pour protéger, hydrater et/ou raffermir la peau.

L'invention a aussi pour objet l'utilisation d'une composition contenant, dans un milieu acceptable pour une application topique, au moins un filtre absorbant au moins le rayonnement ultraviolet A et au moins un agent anti-oxydant et/ou antiradicalaire ne présentant pas de propriété filtrante dans le rayonnement ultraviolet A et conférant à cette composition un indice de protection (IP) dans ce rayonnement ultraviolet A, supérieur à celui du filtre seul, pour lutter contre le vieillissement photo-induit.

L'invention a encore pour objet une utilisation de la composition définie ci-dessus pour dépigmenter la peau et/ou traiter cosmétiquement les taches cutanées dues au vieillissement, présentes sur le visage et/ou le corps, y compris les mains et le cuir chevelu ainsi que pour la préparation d'une crème destinée au traitement des taches de la peau d'origine pathologique.

L'invention se rapporte aussi à l'utilisation d'une composition contenant, dans un milieu acceptable pour une application topique, au moins un filtre absorbant au moins le rayonnement ultraviolet A et au moins un agent anti-oxydant et/ou antiradicalaire ne présentant pas de propriété filtrante dans le rayonnement ultraviolet A et conférant à cette composition un indice de protection (IP) dans ce rayonnement ultraviolet A, supérieur à celui du filtre seul, pour protéger la peau contre les radicaux libres.

L'invention a encore pour objet un procédé de traitement cosmétique et/ou dermatologique de la peau, consistant à appliquer sur la peau une composition contenant, dans un milieu acceptable pour une application topique, au moins un filtre absorbant au moins le rayonnement ultraviolet A et au moins un agent anti-oxydant et/ou antiradicalaire ne présentant pas de propriété filtrante dans le rayonnement ultraviolet A et conférant à cette composition un indice de protection (IP) dans ce rayonnement ultraviolet A, supérieur à celui du filtre seul.

D'autres caractéristiques et avantages de l'invention ressortiront mieux de la description qui suit, donnée à titre illustratif et non limitatif. Dans les exemples ci-après les compositions sont données en % pondéral.

Les tests suivants ont mis en évidence les avantages de la présente invention. Le but des tests a été de montrer l'aptitude des compositions de l'invention à la protection contre les radicaux libres.

A titre d'exemple, on a réalisé la composition (A) suivante :
émulsion huile-dans-eau avec alpha D,L tocophérol + ascorbyl glucoside + caféine + alpha glycosyl rutine + sel penta sodique de l'acide éthylène di-amine tétraméthylène phosphonique (Dequest 2046 de chez Monsanto) + superoxyde

dismutase + acide benzène 1,4 [di(3-méthylidène-campho 10-sulfonique)]. On a aussi réalisé la composition (B) qui est la même émulsion que la composition (A) mais qui ne contient que le filtre UVA.

Sur la figure annexée, on a représenté les courbes d'absorption représentée par la densité optique (D.O) en fonction de la longueur d'onde X, exprimée en nanomètre, de ces deux compositions.

On voit sur ces courbes d'absorption que la composition (A) possède la même absorption dans l'UVA que la composition (B). Autrement dit l'association des actifs antioxydants et/ou anti-radicalaires n'a pas d'effet sur le spectre d'absorption du filtre UVA. Ceci est également prouvé par les valeurs des surfaces UVA des deux compositions.

| | Surface UVA | Surface UVB |
|---|---|---|
| Composition (A) | 431(+/-25) | 267(+/-15) |
| Composition (B) | 460(+/-9) | 156(+/-2) |

En revanche, et de façon surprenante, les indices de protection UVA (IPD) sont supérieurs à celui du filtre seul pour les différentes compositions (A), (C) à (H), ce qui montre que les compositions de l'invention filtrent le rayonnement UVA de façon plus efficace que le filtre seul.

Les valeurs des indices de protection UVA (IP) des compositions (A) et (B) ont été mesurées in vivo sur 15 sujets (méthode PPD), ainsi que celles des compositions suivantes sur 5 ou 10 sujets :

| | |
|---|---|
| Composition (C): | Filtre UVA + alpha D,L tocophérol seul. |
| Composition (D) : | filtre UVA + caféine seule. |
| Composition (E) : | filtre UVA + ascorbyl glucoside seul. |
| Composition (F): | filtre UVA + alpha glycosyl rutine. |
| Composition (G) : | filtre UVA + sel penta sodique de l'acide éthylène di-amine tétra méthylène phosphonique seul (Dequest 2046). |
| Composition (H) : | filtre UVA + superoxyde dismutase. |

Comme indiqué ci-dessus, les IP ont été déterminés selon la méthode PPD. Les tests sont réalisés sur les dos des sujets où sont délimitées des zones cutanées du dos, ayant une surface d'environ 75 $cm^2$. Le produit à tester est appliqué sur certaines zones. 15 minutes après application du produit, toutes les zones (celles ayant reçu du produit et les autres) sont soumises à l'exposition d'un filtre UVA dont la source lumineuse est une lampe à arc xénon de 150 W munie de filtres WG 335 et UG 11, permettant de délivrer des doses de rayonnement ultraviolet de 5 à 38 joules/$cm^2$ en progression géométrique de 50 %.

On détermine la dose pigmentante minimale, c'est-à-dire la dose provoquant la première réaction de pigmentation perceptible sans ambiguité et homogène, sur une zone protégée par le produit à étudier (D.P.M. protégée) et sur une zone où aucune application n'a été réalisée (D.P.M. non protégée).

L'indice de protection est le rapport entre D.P.M. protégée et D.P.M. non protégée :

$$IP=\frac{\text{D.P.M. non protégée}}{\text{D.P.M. non protégée}}$$

Les résultats sont :

Composition (A) > Composition (B) pour 80 % des sujets (25 % d'augmentation).
Composition (A) > Composition (C) pour 60 % des sujets (12 % d'augmentation).
Composition (A) > Composition (D) pour 60 % des sujets (16 % d'augmentation).
Composition (A) > Composition (E) pour 70 % des sujets (47 % d'augmentation).
Composition (A) > Composition (F) pour 60 % des sujets (15 % d'augmentation).
Composition (A) > Composition (G) pour 80 % des sujets (8 % d'augmentation).
Composition (A) > Composition (H) pour 100 % des sujets (10 % d'augmentation).

La protection dans l'UVA de la composition (A) qui regroupe l'association antiradicalaire complète avec le filtre UVA est donc supérieure à celle de la composition (B) contenant le filtre seul. Elle est aussi supérieure à celles des compositions (C) à (H) qui contiennent le filtre UVA associé à un actif d'un seul des groupes mentionnés précédemment. Dans tous les cas la supériorité de la composition (A) est retrouvée pour plus de 50% des sujets.

En outre, les compositions (C), (D), (F), (G) et (H) donnent aussi une protection dans l'UVA supérieure à la composition (B), comme en témoigne les valeurs suivantes.

Composition (C) > Composition (B) pour 40 % des sujets, avec 11% d'augmentation. L'alpha D, L tocophérol entraîne donc une augmentation de protection, supérieure à (B).

Composition (D) > Composition (B) pour 60 % des sujets, avec 7% d'augmentation. La caféine entraîne donc une augmentation de protection, supérieure à la composition (B).

Composition (F) > Composition (B) pour 20 % des sujets, avec 7% d'augmentation. L'alpha glycosyl rutine n'entraîne qu'une faible augmentation de la protection par rapport à la composition (B).

Composition (G) > Composition (B) pour 75 % des sujets, avec 15% d'augmentation. Le chélateur de fer entraîne donc une augmentation notable de la protection, par rapport à (B).

Composition (H) > Composition (B) pour 60 % des sujets, avec 13% d'augmentation. La superoxide dismutase entraîne donc une augmentation de protection, supérieure à la composition (B).

Seule la composition (E) donne une protection dans les UVA, inférieure à la composition (B). Ceci est lié au fait que l'ascorbyl glucoside n'a pas directement de propriétés antioxydantes ; il est utilisé dans l'invention comme régénérateur du radical tocophéryle.

**EXEMPLE 1** : Crème eau-dans-huile anti-rides

**Composition**

*$A_1$*

- Acide stéarique 0,4 %
- Stéarate de polyéthylène glycol (40.OE) (émulsionnant) 3,5 %
- Mono,di,tri-palmitostéarate de glycéryle (émulsionnant) 3,0 %
- Myristate de myristyle (huile) 2,0 %
- Palmitate d'isopropyle (huile) 7,0 %
- Iso-paraffine (6-8 moles d'isobutylène) hydrogénée (huile) 6,5 %

*$A_2$*

- Cyclopentadiméthylsiloxane (huile) 5,0 %

*B*

- Eau déminéralisée stérilisée qsp 100 %
- Glycérol (hydratant) 3,0 %
- Mélange de chélateur de fer, de tocophérol, de SOD, d'ascorbyl glucoside et d'alpha glycosyl rutine 5,0 %
- Alcool éthylique absolu dénaturé 10,0 %
- Para-hydroxybenzoate de méthyle (conservateur) 0,2 %

C

- Acide téréphtalylidène di-camphosulfonique dans l'eau à 33 % 3,3 %
- Triéthanolamine (neutralisant) 0,7 %

**Préparation de la phase $A_1$ + $A_2$**

Les constituants de $A_1$ sont solubilisés à 80 °C. Lorsque le mélange est limpide, la température est abaissée à 65 °C et on ajoute $A_2$. Le mélange doit être limpide et homogène. On maintient la température de 65 °C.

**Fabrication**

Dans un bécher de fabrication, les constituants de B sont solubilisés à 85 °C-90 °C. Après vérification de la limpidité, la température est ramenée à 65 °C. On réalise l'émulsion, sous agitation, en versant ($A_1$+$A_2$) dans B. On poursuit le refroidissement sous agitation. A 40 °C on ajoute la phase C, toujours sous agitation, et on laisse refroidir à 20 °C sous agitation.

On obtient une crème blanche de soin pour protéger quotidiennement la peau des méfaits du rayonnement UV et empêcher la formation des rides et ridules photo-induites.

**EXEMPLE 2** : Gel protecteur contre les rayons solaires

**Composition**

*A*

- Eau déminéralisée qsp 100 %
- Glycérine 3,0 %
- Para-hydroxybenzoate de méthyle 0,2 %
- Dequest 2046 + Vitamine E 1,0%
- Gomme de xanthane (épaississant) 0,2 %

*B*

- Parsol MCX (filtre UVB) 4,0 %
- Alkyl benzoate (Finsov TN, société Witco) 4,0 %
- Carbomer (Pemulen TR 2, société Goodrich) 0,5 %
- Triéthanolamine 0,5 %

*C*

- Acide téréphtalylidène di-camphosulfonique dans l'eau à 33% 2,3 %
- Triéthanolamine (neutralisant) 0,7 %

**Fabrication**

On prépare la phase A en saupoudrant, sous agitation le gélifiant dans l'eau contenant les ingrédients dissous. On émulsionne en incorporant la phase B à la phase A, sous agitation. On lisse et on laisse refroidir sous agitation à pales lentes. A 35 °C, on ajoute la phase C et on laisse refroidir à 25 °C, toujours sous agitation.

**EXEMPLE 3:** Crème de jour protectrice (émulsion huile-dans-eau)

Composition

*$A_1$*

- Stéarate de glycéryle 2,0 %
- PEG-150 distéarate (émulsionnant) 1,0 %
- Alcool cétylique 2,0 %
- Polysorbate 40 (émulsionnant) 0,5 %
- Polyisobutène hydrogéné (huile) 15,0 %
- Cyclopentadiméthylsiloxane (huile) 4,0 %
- Polyacrylamide/$C_{13}$-$C_{14}$ Isoparaffin/Laureth-7 (Sepigel 305 vendu par la société Seppic) (épaississant) 0,5 %

*B*

- Dioxyde de titane (nanopigments traités par alumine-silice et vendus sous la dénomination « M212 » par la société Kemira) 2,0 %
- Glycérine (hydratant) 4,0 %

*C*

- Dequest 2046 (chélateur) 0,1 %
- Extrait de thé vert (« camelia sinensis extract » vendu par la société Indena) 0,05 %
- SOD[Cu-Zn] (SOD d'origine végétale vendue par la société Silab) 0,01 %
- Para-hydroxybenzoate de méthyle (conservateur) 0,2 %
- Eau déminéralisée stérilisée qsp 100 %

Fabrication

Les constituants de A sont homogénéisés à 70°C, ceux de B et ceux de C à la température ambiante. On réalise l'émulsion, sous agitation, en versant B dans A à 70°C. On refroidit sous agitation. A 40°C on ajoute la phase C, toujours sous agitation, et on laisse refroidir jusqu'à la température ambiante sous agitation.

On obtient une crème blanche de soin pour protéger quotidiennement la peau des méfaits du rayonnement UV.

## Revendications

1. Utilisation d'au moins un agent anti-oxydant et/ou antiradicalaire ne présentant pas de propriété filtrante dans le rayonnement ultraviolet A, pour conférer à une composition contenant, dans un milieu acceptable pour une application topique, au moins un filtre absorbant au moins le rayonnement ultraviolet A, un indice de protection (IP) dans ce rayonnement ultraviolet A, supérieur à celui du filtre seul.

2. Utilisation selon la revendication 1, caractérisée en ce que l'agent est un chélateur de fer.

3. Utilisation selon la revendication 1 ou 2, caractérisée en ce que la composition contient au moins un autre agent anti-oxydant et/ou antiradicalaire, choisi parmi les agents antilipo-peroxyde, les composés régénérateurs de vitamine E oxydé, les agents antiradical hydroxyle, les agents anti-oxygène singulet et les agents antiradical anion supersoxyde et leurs associations.

4. Utilisation selon l'une des revendications précédentes, caractérisée en ce que le filtre représente de 0,01% à 10% et de préférence de 0,1% à 5% du poids total de la composition.

5. Utilisation selon l'une des revendications précédentes, caractérisée en ce que le filtre est choisi parmi les filtres organiques et les pigments.

6. Utilisation selon l'une des revendications précédentes, caractérisée en ce que le filtre est choisi parmi les pigments de titane ou de zinc.

7. Composition selon la revendication précédente, caractérisée en ce que les pigments sont des nanopigments.

8. Utilisation selon l'une des revendications précédentes, caractérisée en ce que le filtre est choisi parmi les filtres organiques comportant au moins une fonction sulfonique éventuellement neutralisée.

9. Utilisation selon l'une des revendications précédentes, caractérisée en ce que le filtre est un dérivé sulfoné ou sulfonaté du benzylidène camphre.

10. Utilisation selon l'une des revendications précédentes, caractérisée en ce que le filtre présente la formule (I) suivante :

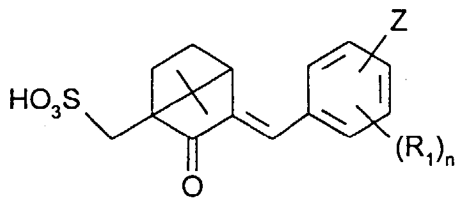

(I)

dans laquelle :

- Z désigne un groupement

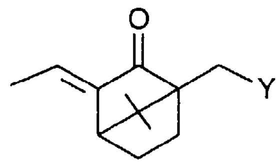

dans lequel Y représente -H ou -$SO_3H$, éventuellement neutralisé,

- n est égal à 0 ou est un nombre allant de 1 à 4 ($0 \leq n \leq 4$),
- $R_1$, représente un ou plusieurs radicaux alkyle ou alkoxy, identiques ou différents, linéaires ou ramifiés, contenant de 1 à 4 atomes de carbone.

**11.** Utilisation selon l'une des revendications précédentes, caractérisée en ce que le filtre est l'acide benzène 1,4 [di (3-méthylidène campho-10-sulfonique)].

**12.** Utilisation selon l'une des revendications précédentes, caractérisée en ce que la composition se présente sous forme d'une émulsion, d'un gel ou sous forme d'une dispersion de sphérules.

**13.** Utilisation selon l'une des revendications 2 à 12, caractérisée en ce que le chélateur de fer présente une constante d'association avec les ions ferreux ou ferriques supérieure à $10^2$ et de préférence supérieure à $10^6$.

**14.** Utilisation selon l'une des revendications 2 à 13, caractérisée en ce que le chélateur de fer représente de 0,001 % à 5 % et mieux de 0,1 % à 2 % du poids total de la composition.

**15.** Utilisation selon l'une des revendications 3 à 14, caractérisée en ce que l'agent antiliperoxyde représente de 0,05 % à 5 % et mieux de 0,2 % à 3 % du poids total de la composition.

**16.** Utilisation selon l'une des revendications 3 à 15, caractérisée en ce que le composé régénérateur de vitamine E représente de 0,01 % à 5 % et de préférence de 0,1 % à 1 % du poids total de la composition.

**17.** Utilisation selon l'une des revendications 3 à 16, caractérisée en ce que l'agent antiradical hydroxyle représente de 0,001 % à 5 % et mieux de 0,005 % à 2 % du poids total de la composition.

**18.** Utilisation selon l'une des revendications 3 à 17, caractérisée en ce que l'agent anti-oxygène singulet représente de 0,0001 % à 1 % et mieux de 0,0005 % à 1 % du poids total de la composition.

**19.** Utilisation selon l'une des revendications 3 à 18, caractérisée en ce que l'agent antiradical anion superoxyde représente de 0,1 % à 5 % et mieux de 0,5 % à 3 % du poids total de la composition.

**20.** Utilisation selon l'une des revendications 3 à 19, caractérisée en ce que la composition contient, en association, un filtre UVA, un chélateur de fer et un agent antilipo-peroxyde.

**21.** Utilisation selon l'une des revendications 3 à 20, caractérisée en ce que la composition contient, en association, un filtre absorbant au moins le rayonnement ultraviolet A, un composé régénérateur de vitamine E oxydé et un agent antilipo-peroxyde.

**22.** Utilisation selon l'une des revendications 3 à 21, caractérisée en ce que la composition contient, en association, de l'alpha D, L tocophérol, un sel penta sodique de l'acide éthylène di-amine tétraméthylène phosphonique et de l'acide benzène 1,4 [di(3-méthylidène-campho 10-sulfonique)].

**23.** Utilisation selon l'une des revendications 3 à 22, caractérisée en ce que la composition contient, en association, l'acide benzène 1,4 [di(3-méthylidène-campho 10-sulfonique)], de la vitamine E ou un de ses dérivés, de la vitamine C ou un de ses dérivés.

**24.** Utilisation selon l'une des revendications 3 à 23, caractérisée en ce que la composition contient, en association, de l'alpha D, L tocophérol, de l'ascorbyl glucoside, de la caféine, de l'alpha glycosyl rutine, un sel penta sodique

de l'acide éthylène di-amine tétraméthylène phosphonique, de la superoxyde dismutase et de l'acide benzène 1,4 [di(3-méthylidène-campho 10-sulfonique)].

**25.** Utilisation d'une composition contenant, dans un milieu acceptable pour une application topique, au moins un filtre absorbant au moins le rayonnement ultraviolet A et au moins un agent anti-oxydant et/ou antiradicalaire ne présentant pas de propriété filtrante dans le rayonnement ultraviolet A et conférant à cette composition un indice de protection (IP) dans ce rayonnement ultraviolet A, supérieur à celui du filtre seul, pour protéger la peau contre les radicaux libres.

**26.** Utilisation d'une composition contenant, dans un milieu acceptable pour une application topique, au moins un filtre absorbant au moins le rayonnement ultraviolet A et au moins un agent anti-oxydant et/ou antiradicalaire ne présentant pas de propriété filtrante dans le rayonnement ultraviolet A et conférant à cette composition un indice de protection (IP) dans ce rayonnement ultraviolet A, supérieur à celui du filtre seul, pour lutter contre le vieillissement photo-induit.

**27.** Utilisation d'une composition contenant, dans un milieu acceptable pour une application topique, au moins un filtre absorbant au moins le rayonnement ultraviolet A et au moins un agent anti-oxydant et/ou antiradicalaire ne présentant pas de propriété filtrante dans le rayonnement ultraviolet A et conférant à cette composition un indice de protection (IP) dans ce rayonnement ultraviolet A, supérieur à celui du filtre seul, pour lutter contre les rides et/ou les ridules de la peau induites par un rayonnement ultraviolet A.

**28.** Procédé de traitement cosmétique de la peau, caractérisé en ce qu'il consiste à appliquer sur la peau une composition contenant, dans un milieu acceptable pour une application topique, au moins un filtre absorbant au moins le rayonnement ultraviolet A et au moins un agent anti-oxydant et/ou antiradicalaire ne présentant pas de propriété filtrante dans le rayonnement ultraviolet A et conférant à cette composition un indice de protection (IP) dans ce rayonnement ultraviolet A, supérieur à celui du filtre seul.

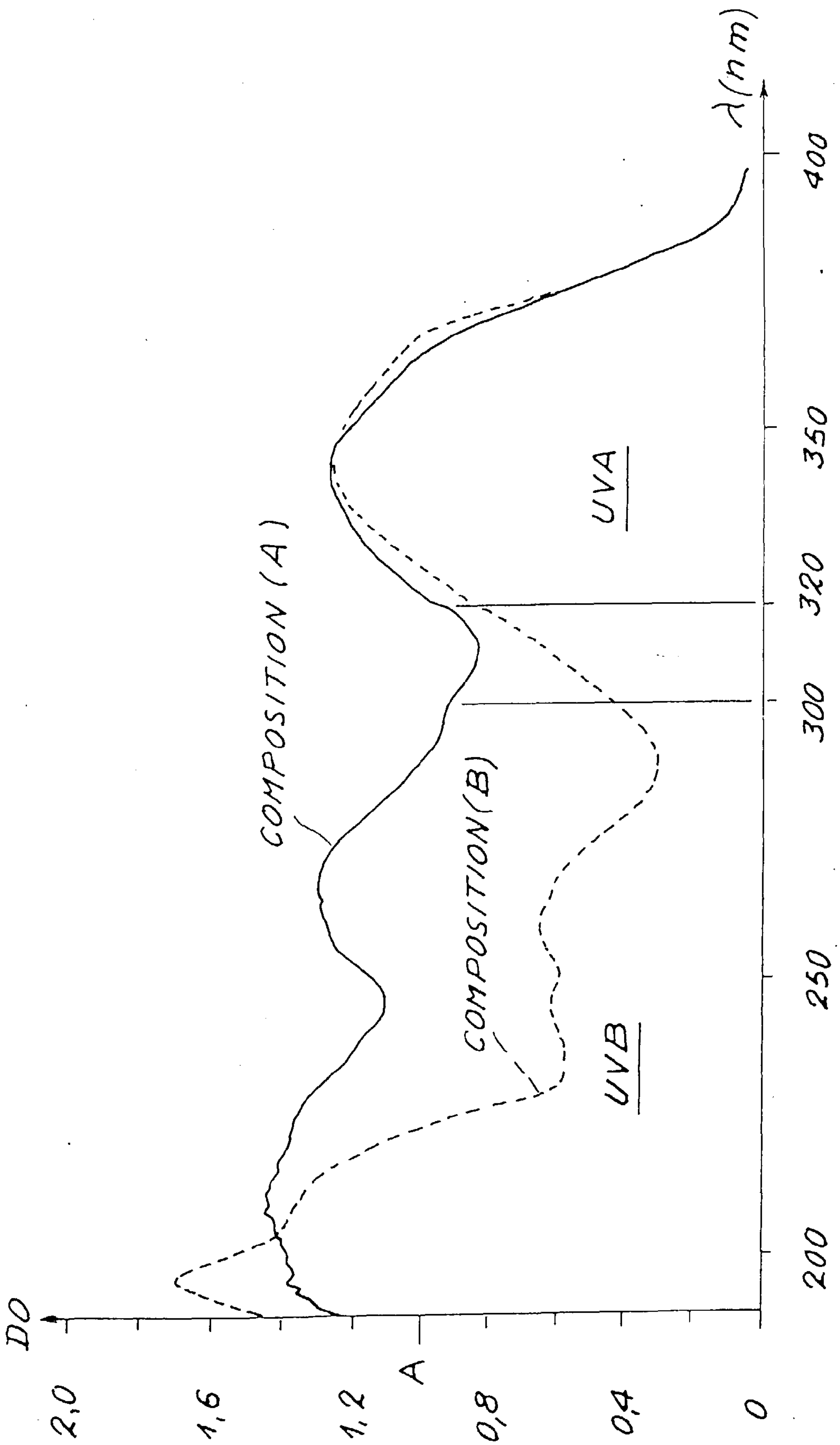
DO
2,0
1,6
1,2
A
0,8
0,4
0
200
250
300
320
350
400
λ(nm)
COMPOSITION (A)
COMPOSITION (B)
UVB
UVA